# EUROPÄISCHE PATENTSCHRIFT

(11) **EP 0 504 584 B1**
(45) Veröffentlichungstag und Bekanntmachung des Hinweises auf die Patenterteilung: **26.10.1994**
(21) Anmeldenummer: 92102294.3
(22) Anmeldetag: 12.02.1992
(51) Int. Cl.: A61B 17/068

(54) **Gerät zum Setzen von Wundklammern**
Surgical fastener apparatus
Agrafeuse chirurgicale

(30) Priorität: 19.03.1991 DE 4108952
(43) Veröffentlichungstag der Anmeldung: 23.09.1992
(73) Patentinhaber: Beiersdorf Aktiengesellschaft, 20245 Hamburg (DE)
(72) Erfinder: Heimerl, Albert, Dr., W-2000 Hamburg 65 (DE); Kartheus, Holger, W-2000 Hamburg 20 (DE); Paske, Dietmar, W-2150 Buxtehude (DE); Plenio, Hans-Ulrich, Dr., W-2100 Hamburg 90 (DE)
(74) Vertreter: Wilcken, Thomas, Dipl.-Ing.

(56) Entgegenhaltungen:
- EP-A- 0 244 854
- EP-A- 0 423 589

## Beschreibung

Die Erfindung betrifft ein Gerät zum Setzen von Wundklammern, bestehend aus zwei mittels einer Verbindung lösbar verbundenen Geräteteilen, wobei einem ersten Geräteteil ein Magazin mit den Wundklammern und einem zweiten Geräteteil ein feststehender Griff und ein hierzu verstellbarer Griff zum Betätigen des Gerätes zugeordnet sind.

Mit der zunehmenden Anwendung von Wundklammergeräten anstelle der klassischen Methode des Wundverschlusses mit Nadel und Faden entstehen für den Anwender höhere Produktkosten, so daß die Hersteller solcher Geräte nach Möglichkeiten suchen, die Kosten für die Herstellung und spätere Verwendung dieser Geräte niedrig zu halten. Mit herkömmlichen Wegwerfgeräten, die also nicht für eine Wiederverwendung nach Sterilisation vorgesehen sind, ist dieses Ziel allerdings nicht zu erreichen.

Man ist deshalb dazu übergegangen, nur einen Teil des Gerätes als Wegwerfteil auszubilden, der hinsichtlich seiner Bauelemente auf das unter dem Aspekt der Funktion und Hygiene notwendige Maß eingeschränkt ist. Ein gerät zum Setzen von Wundklammern nach dem Oberbegriff des Anspruchs 1 ist aus der EP-A-244 854 bekannt. Solche Geräte (DE-A-39 34 698, EP-A- 244 854) bestehen dann aus einem ersten nicht wiederverwendbaren Geräteteil mit dem die Wundklammern aufnehmenden Magazin und einem zweiten Geräteteil mit den resterilisierbaren Handgriffen und sonstigen Teilen. Dieses sogenannte Semi-Disposable-Konzept wirkt sich beim Verwender nicht allein auf der Kostenseite positiv aus, es ist auch unter dem Gesichtspunkt des Umweltschutzes von Vorteil.

Die lösbare Verbindung zwischen beiden Geräteteilen muß beispielsweise bei einem erforderlichen Magazinwechsel leicht vom Operateur hergestellt werden können, da während einer Operation eine unkomplizierte Handhabung des Gerätes gewünscht wird. Andererseits erfordert die Anwendung des Gerätes am Patienten eine absolut sichere Verbindung zwischen beiden Geräteteilen.

Es hat sich aber in der Praxis gezeigt, daß eine üblicherweise verwendete Rast- oder Schnappverbindung zwischen beiden Geräteteilen eine nur geringe Haltekraft haben kann, wenn man die Bedingung erfüllen will, daß sich beide Geräteteile leicht zusammenstecken und auch wieder voneinander lösen lassen. Eine solche kraftschlüssige Verbindung wird deshalb meist nur geeignet sein, ein unbeabsichtigtes Ablösen des das Magazin aufnehmenden Geräteteils in der Ruhephase sicher zu verhindern. Dies läßt sich aber nicht unbedingt auch während der Arbeitsphase gewährleisten, also während der Betätigung des Gerätes. Hierbei entstehen nämlich durch das Zusammendrücken der Griffe Kräfte, die auf das Magazin, die Werkzeuge zum Verformen der Wundklammer und sonstige im ersten Geräteteil befindliche Bauelemente einwirken und so stark werden können, daß die ihnen entgegenwirkende Haltekraft einer Schnappverbindung überwunden wird und beide Geräteteile ungewollt getrennt werden.

Falls das Magazin und der wiederverwendbare Geräteteil mittels einer lösbaren Rastverbindung über eine Rastklinke formschlüssig verbunden werden (EP-A-0 244 854), wird ein nicht beabsichtigtes Lösen des Magazins zu vermeiden sein, wenn die Verbindungsmittel entsprechend stabil ausgelegt werden. Im Gegensatz zu einer Schnappverbindung hat eine solche Rastverbindung aber den Nachteil eines relativ hohen baulichen Aufwandes und einer umständlichen Handhabung, da die Rastverbindung jeweils von Hand aufgehoben werden muß, bevor das Magazin entnommen werden kann.

Durch die Erfindung sollen diese beschriebenen Nachteile bekannter Geräte beseitigt werden. Insbesondere soll ein Wundklammergerät vorgeschlagen werden, bei dem einerseits sich beide Geräteteile einfach und ohne großen Kraftaufwand während der Ruhephase miteinander verbinden und voneinander trennen lassen und andererseits trotzdem gewährleistet ist, daß eine Trennung der Geräteteile während der Betätigungs- bzw. Arbeitsphase sicher verhindert wird.

Zur Lösung dieser Aufgabe ist das eingangs erwähnte Gerät gekennzeichnet durch eine neben der erwähnten ersten Verbindung vorgesehene zweite und nur während der Betätigungsphase wirksame Verbindung zwischen beiden Geräteteilen.

Hierdurch ergibt sich neben der Haltekraft der ohnehin vorhandenen ersten lösbaren Verbindung, die beispielsweise durch einen Schnappmechanismus hergestellt wird, eine zusätzliche Haltekraft durch eine zweite Verbindung, die allein während der Betätigung des Gerätes wirksam wird und im übrigen unwirksam ist, wenn das Gerät unbetätigt in der Ruhephase ist.

Durch entsprechende funktionelle und bauliche Auslegung der weiteren Verbindung läßt sich erreichen, daß während der Arbeitsphase ein Ablösen des ersten Geräteteiles vom zweiten Geräteteil sicher vermieden wird. Im übrigen ergibt sich der weitere Vorteil, daß die erste lösbare Verbindung nur so stark ausgelegt werden muß, daß sie eine unbeabsichtigte Trennung der Geräteteile während der Ruhephase verhindert und ein beabsichtigtes Trennen einfach und ohne besonderen Kraftaufwand ermöglicht. Die erwähnte zweite Verbindung kann beispielsweise durch einen zeitweilig wirksamen Formschluß zwischen beiden Geräteteilen gebildet werden Wenn es um ein Gerät geht, bei dem der verstellbare Griff relativ zum anderen Griff um eine Achse verschwenkbar ist, sollten die den Formschluß bildenden Teile um diese Achse verlaufen, so daß die Möglichkeit besteht, diese Teile einerseits am verstellbaren Griff und andererseits an einem relativ hierzu feststehenden Geräteteil anzuordnen. Wenn der verstellbare Griff durch den längeren Hebelarm eines doppelarmigen Hebels gebildet ist, dessen anderer kürzerer Hebelarm zur Verstellung eines die Klammern verformenden Werkzeuges dient, können dementsprechend die den Formschluß herstellenden Teile zum einen am kürzeren Hebelarm und zum anderen am ersten Geräteteil angeordnet werden. In diesem Fall bietet es sich insbesondere an, die Formschlußteile an den beiden Längsseiten des kürzeren Hebelarmes und den beiden gegenüberliegenden Seitenwänden des Gehäuses des ersten Geräteteiles vorzusehen.

Eine formschlüssige und nur zeitweilig während der Arbeitsphase wirksame Verbindung beider Geräteteile läßt sich auch erreichen, wenn man diese mit einem durch Betätigung der Griffe verstellbaren Stift, Riegel oder Haken miteinander verrastet, verriegelt oder verhakt. Auch sind zu diesem Zweck kraftschlüssige Verbindungen mit Klemm- oder Spreizelementen möglich.

Die Erfindung wird nachfolgend anhand eines in der anliegenden Zeichnung dargestellten Ausführungsbeispieles naher erläutert. Es zeigt:
- Figur 1: eine perspektivische Seitenansicht eines Gerätes nach der Erfindung,
- Figur 2: einen teilweisen längsschnitt durch das Gerät nach Figur 1 und
- Figur 3: einen Schnitt entsprechend der Schnittlinie III-III in Fig.2.

Das Gerät besteht aus zwei Geräteteilen 1 und 2, deren Gehäuse an der Trennebene 3 gegeneinander anliegen. Im ersten auswechselbaren Geräteteil 1 sind die Wundklammern mit dem sie aufnehmenden Klammerträger, die Werkzeuge zum Verformen und Implantieren einer Klammer und sonstige Teile untergebracht, die bekannt sind (EP-A-0 244 854, WO-A-82/02 486, US-A-4 109 844) und deshalb nicht im einzelnen dargestellt und beschrieben werden müssen.

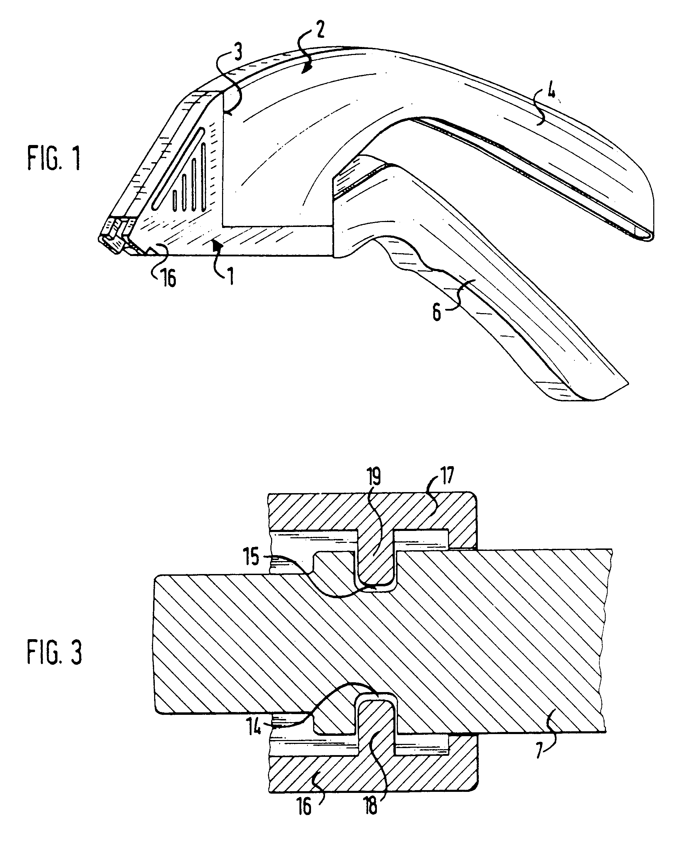

Zum resterilisierbaren zweiten Geräteteil 2 gehören ein feststehender Griff 4 und ein hierzu um eine Achse 5 verschwenkbarer Griff 6, der Teil eines doppelarmigen Hebels ist und dessen kürzerer Hebelarm 7 mit seinem freien Ende auf die im Geräteteil 1 befindlichen Werkzeuge zum Halten und Verformen der Wundklammern einwirkt. Von diesen Werkzeugen ist in der Figur 1 nur ein Teil 8 zu sehen.

Ain Gehäuse des Geräteteiles 1 befindet sich ein Ansatz 9 mit einer Nase 10, die beim Zusammenstecken der Geräteteile in eine Ausnehmung 11 schnappt, die in einer Wand 12 der im Geräteteil 2 ausgebildeten Aufnahme 13 vorgesehen ist. Es handelt sich hierbei um die schon vorher erwähnte Schnappverbindung, die bei diesem Ausführungsbeispiel als lösbare erste Verbindung der Geräteteile dient. Erwähnt sei an dieser Stelle, daß auch mehrere Verbindungen und ebenfalls andere Verbindungsarten möglich und anwendbar sind, wobei vorzugsweise Verbindungen in Betracht kommen werden, die beim Fügen oder Trennen der Geräteteile selbsttätig wirksam bzw. aufgehoben werden.

Man kann sich vorstellen, daß bei Betätigung des Gerätes durch Verschwenken des Griffes 6 in Richtung auf den Griff 4 beträchtliche Kräfte an der Stelle wirksam werden, wo der Hebelarm 7 auf die Werkzeuge 8 einwirkt, und daß dies zu einer entsprechend hohen Belastung der vorher beschriebenen Schnappverbindung führen wird. Jedenfalls muß je nach Auslegung dieser Verbindung damit gerechnet werden, daß sie aufgrund der an ihr auftretenden Schub- und Kippkräfte gelöst wird und die Geräteteile während der Arbeitsphase unbeabsichtigt getrennt werden. Dies verhindert nun aber eine weitere und nur während der Betätigungsphase wirksam werdende zweite Verbindung.

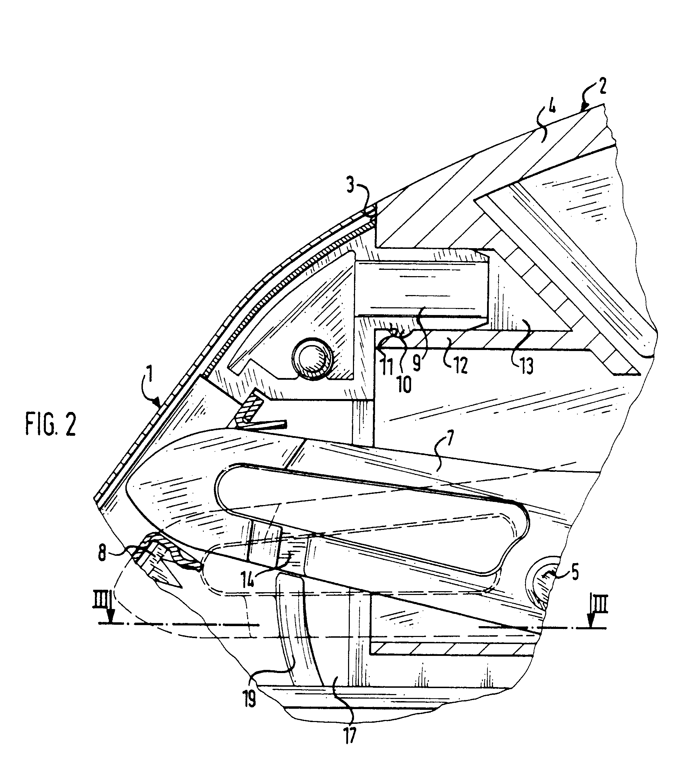

Hierfür zeigen die Figuren 2 und 3 eine praktische Ausführungsform. An den beiden vertikalen Seiten des Hebelarmes 7 sind Ausnehmungen 14, 15 und an den beiden gegenüberliegenden Wänden 16, 17 des Geräteteiles 1 Vorsprünge 18, 19 vorgesehen, die jeweils auf einem Kreisbogen bzw. gleichen Radius um die Achse 5 verlaufen.

Die Figur 2 zeigt das Gerät und somit auch den mit ausgezogenen Linien dargestellten Hebelarm 7 während der Ruhephase. Bei Betätigung des Gerätes wird der Hebelarm 7, bezogen auf diese Darstellung, nach unten verschwenkt, wobei die Ausnehmungen 14, 15 und die Vorsprünge 18, 19 nach Art einer Nut-Feder-Verbindung in gegenseitigen Eingriff gelangen, indem die Vorsprünge je nach fortschreitender Verschwenkung des Hebelarmes weiter in und durch die Ausnehmungen greifen, bis schließlich die mit unterbrochenen Linien in Figur 2 gezeigte Stellung des Hebelarmes 7 erreicht ist.

Die hierdurch geschaffene formschlüssige Verbindung, die beide Geräteteile 1, 2 während der Arbeitsphase sicher zusammenhält, ist wieder aufgehoben, sobald der Griff 6 nach Implantation einer Wundklammer wieder entlastet und in seine Ruheposition zurückgekehrt ist, bei der sich dann natürlich auch der Hebelarm 7 wieder in seiner Ausgangsposition befindet. Bei dieser Position können beide Geräteteile bei Bedarf und unbehindert durch die nur vorher wirksam gewesene zweite Verbindung leicht voneinander getrennt werden, indem der Geräteteil 1, gemäß der Darstellung nach Figur 2 nach links vom Geräteteil 2 abgezogen wird, wobei nur verhältnismäßig geringe Kräfte zum Lösen der ersten Verbindung zwischen den Teilen 9, 10 und 11, 12 aufzuwenden sind.

## Patentansprüche

1. Gerät zum Setzen von Wundklammern, bestehend aus zwei mittels einer Verbindung (9, 10) lösbar verbundenen Geräteteilen (1, 2), wobei dem ersten Geräteteil (1) zumindest ein Magazin mit den Klammern und dem zweiten Geräteteil (2) ein feststehender Griff (4) und ein hierzu verstellbarer Griff (6) zum Betätigen des Gerätes zugeordnet sind, gekennzeichnet durch eine neben der vorerwähnten ersten Verbindung (9, 10) vorgesehene zweite und nur während der Betätigungsphase wirksame Verbindung (14, 15, 18, 19) zwischen beiden Geräteteilen (1, 2).

2. Gerät nach Anspruch 1, dadurch gekennzeichnet, daß die erwähnte zweite Verbindung (14, 15, 18, 19) durch einen Formschluß zwischen beiden Geräteteilen (1, 2) gebildet wird.

3. Gerät nach den Ansprüchen 1 und 2, bei dem der verstellbare Griff (6) relativ zum anderer Griff (4) um eine Achse (5) verschwenkbar ist, dadurch gekennzeichnet, daß die den Formschluß bildenden Teile (14, 15, 18, 19) jeweils auf einem Kreisbogen um die Schwenkachse (5) liegen.

4. Gerät nach einem der Ansprüche 1 bis 3, bei dem der verstellbare Griff (6) durch den längeren Hebelarm eines doppelarmigen Hebels gebildet ist, dessen anderer kürzerer Hebelarm (7) zur Verstellung eines die Klammern verformenden Werkzeuges (8) dient, dadurch gekennzeichnet, daß den Formschluß herstellende Teile (14, 15, 18, 19) einerseits am kürzeren Hebelarm (7) und andererseits am ersten Geräteteil (1) angeordnet sind.

5. Gerät nach Anspruch 4, dadurch gekennzeichnet, daß den Formschluß bildende Teile (14, 15, 18, 19) an zwei Längsseiten des kürzeren Hebelarmes (7) und den beiden gegenüberliegenden Seitenwänden (16, 17) des Gehäuses des ersten Geräteteiles (1) vorgesehen sind.

6. Gerät nach einem der Ansprüche 2 bis 5, dadurch gekennzeichnet, daß der Formschluß durch eine Verriegelung der beiden Geräteteile (1, 2) gebildet wird.

7. Gerät nach einem der Ansprüche 1 bis 6, dadurch gekennzeichnet, daß die lösbare erste Verbindung (9, 10, 11, 12) zwischen beiden Geräteteilen (1, 2) als Schnappverbindung ausgebildet ist.

8. Gerät nach Anspruch 7, dadurch gekennzeichnet, daß an einem Geräteteil (1) ein Ansatz (9) und am anderen Geräteteil (2) eine Aufnahme (13) für den Ansatz vorgesehen ist und daß der Ansatz und die Aufnahme über die erwähnte Schnappverbindung miteinander verbunden sind.

## Claims

1. Apparatus for placing wound clamps comprising two apparatus parts (1, 2) releasably connected by means of a connection (9, 10), at least one magazine with the clamps being assigned to the first apparatus part (1) and a fixed handle (4) and a handle (6) which can be adjusted with respect to it being assigned to the second apparatus part (2) to actuate the apparatus, characterised by a second connection (14, 15, 18, 19), only effective during the actuation phase and provided in addition to the aforementioned first connection (9, 10), between the two apparatus parts (1, 2).

2. Apparatus according to claim 1, characterised in that the second connection (14, 15, 18, 19) mentioned is formed by a positive lock between the two apparatus parts (1, 2).

3. Apparatus according to claims 1 and 2, in which the adjustable handle (6) can be pivoted relative to the other handle (4) about an axis (5), characterised in that the parts (14, 15, 18, 19) forming the positive lock each lie on an arc of a circle about the pivoting axis (5).

4. Apparatus according to one of claims 1 to 3, in which the adjustable handle (6) is formed by the longer lever arm of a double-armed lever, the other shorter lever arm (7) of which serves for adjusting an implement (8) shaping the clamps, characterised in that parts (14, 15, 18, 19) producing the positive lock on one side are arranged on the shorter lever arm (7) and on the other side are arranged on the first apparatus part (1).

5. Apparatus according to claim 4, characterised in that parts (14, 15, 18, 19) forming the positive lock are provided on two longitudinal sides of the shorter lever arm (7) and the two opposing side walls (16, 17) of the housing of the first apparatus part (1).

6. Apparatus according to one of claims 2 to 5, characterised in that the positive lock is formed by locking the two apparatus parts (1, 2).

7. Apparatus according to one of claims 1 to 6, characterised in that the releasable first connection (9, 10, 11, 12) is designed as a snap connection between two apparatus parts (1, 2).

8. Apparatus according to claim 7, characterised in that a projection (9) is provided on one apparatus part (1) and a recess (13) for the projection is provided on the other apparatus part (2), and in that the projection and the recess are connected to one another by means of the snap connection mentioned.

## Revendications

1. Appareil pour la pose d'agrafes chirurgicales, constitué par deux parties d'appareil (1,2) reliées de façon amovible au moyen d'une liaison (9,10), et dans lequel au moins un magasin contenant les agrafes est associé à la première partie (1) de l'appareil, et une poignée fixe (4) et une poignée déplaçable (6) par rapport à la précédente, pour l'actionnement de l'appareil sont associées à la seconde partie (2) de l'appareil, caractérisé par une seconde liaison (14,15,18,19) prévue en plus de la première liaison (9,10) mentionnée précédemment et agissant uniquement pendant la phase d'actionnement, entre les deux parties (1,2) de l'appareil.

2. Appareil selon la revendication 1, caractérisé en ce que la seconde liaison mentionnée (14,15,18,19) est formée par une liaison de complémentarité de formes entre les deux parties (1,2) de l'appareil.

3. Appareil selon les revendications 1 et 2, dans lequel la poignée déplaçable (6) peut basculer par rapport à l'autre poignée (4) autour d'un axe (5), caractérisé par le fait que les parties (14,15,18,19), qui constituent la liaison par complémentarité de formes, sont situées respectivement sur un arc de cercle autour de l'axe de basculement (5).

4. Appareil selon l'une des revendications 1 à 3, dans lequel la poignée déplaçable (6) est formée par le bras le plus long d'un levier à deux bras, dont l'autre bras plus court (7) sert à déplacer un outil (8) de mise en forme des agrafes caractérisé en ce que les parties (14,15,18,19), qui établissent la liaison par complémentarité de formes, sont disposées d'une part sur le bras le plus court (7) du levier et d'autre part sur la première partie (1) de l'appareil.

5. Appareil selon la revendication 4, caractérisé en ce que les parties (14,15,18,19) qui forment la liaison par complémentarité de formes, sont prévues sur deux côtés longitudinaux du bras le plus court (7) du levier et sur les deux parois latérales opposées (16,17) du boitier de la première partie (1) de l'appareil.

6. Appareil selon l'une des revendications 2 à 5, caractérisé en ce que la liaison par complémentarité de formes est formée par un verrouillage de deux parties (1,2) du boitier.

7. Appareil selon l'une des revendications 1 à 6, caractérisé en ce que la première liaison amovible (9,10,11,12) entre les deux parties (1,2) de l'appareil est réalisée sous la forme d'une liaison à encliquetage.

8. Appareil selon la revendication 7, caractérisé en ce qu'une partie saillante (9) est prévue sur une partie (1) de l'appareil et qu'un logement (13) pour la partie saillante est prévu sur l'autre partie (2) de l'appareil et en ce que la partie saillante et le logement sont reliés entre eux par l'intermédiaire de la liaison à encliquetage mentionnée.
